# EUROPEAN PATENT APPLICATION

(11) **EP 1 291 011 A1**
(43) Date of publication of application: **12.03.2003**
(21) Application number: 00959055.5
(22) Date of filing: 23.06.2000
(51) Int. Cl.: A61K 7/48

(54) **HAND TREATMENT LOTION**

(30) Priority: 14.06.2000 RU 2000114998
(71) Applicant: Uspenskaya, Svetlana Igorevna, Moscow, 117036 (RU)
(72) Inventor: USPENSKAYA, Svetlana Igorevna, Moscow, 117036 (RU); LEONIDOV, Nikolai Borisovich, Moscow, 115407 (RU); SEREGINA, Olga Borisovna, Moscow, 117647 (RU); SAFENINA, Lyudmila Alexandrovna, Moscow, 117602 (RU)
(74) Representative: Goddar, Heinz J., Dr.
(86) International application number: RU0000246
(87) International publication number: WO01095873

(57) **Abstract**

The present invention relates to medicine, particularly to cosmetic industry, and still more particularly to the production of lotions for hand skin care.

It is an object of the invention to enhance the therapeutic-cosmetic properties of the lotion.

Said object is accomplished by a balanced composition of the lotion which provides softening, cleaning, protective, and tonic action, enables maximum penetration of biologically active components into the skin even at low concentrations of the biologically active substances in the lotion, allows them to act on the skin without causing irritating and allergic effect.

The lotion comprises glycerin, ethyl alcohol, sodium hydrocarbonate, water, biologically active components (in particular, plantain sap and/or kalanchoe sap, and/or liquid extract of eleutherococcus, and/or freshly squeezed lemon juice, and/or pantocrine, and/or aloe sap, and/or hypericum tincture, and/or calendula tincture, and/or freshly squeezed apple juice) and essential oils in definite ratios.

Owing to its low allergenicity, the lotion can be used several times a day and, if required, after each contact with water. For individuals with sensitive skin the lotion makes it possible to preserve the tenderness and comfort of the hand skin, protecting it from harmful environmental effects.

## Description

### Field of the Art

The present invention relates to medicine, particularly to cosmetic industry, and still more particularly to the production of therapeutic-cosmetic lotions.

### Background of the Invention

Known in the art is a lotion for skin care, comprising glycerin, ethyl alcohol, vegetable extracts, a perfume, and water (RU 2085186). This lotion features antiseptic, wound-healing and disinfecting effect. However, said lotion is intended for face skin care and contains a small amount of glycerin. This rules out the provision of a protective film required for hand skin care. Besides said lotion does not have sufficient cleaning activity, and a high content of extractive substances may lead to allergic reactions or irritation of the hand skin, taking into account that usually a larger amount of the lotion is applied to the hand skin than to the face.

Known in the art is a hand skin emollient (FS 42-2108-97). This means for external use comprises glycerin, a solution of ammonia, ethyl alcohol, and water. The known lotion provides sufficient cleaning, softening and protective action owing to a large content of glycerin and ammonia. However, said formulation suffers from a number of disadvantages.

The presence of ammonia solution in the formulation leads to the appearance of an unpleasant odor and may cause irritation of the mucous membranes of the eyes and naso-pharynx, while a large amount of glycerin is responsible for the formation of an excessively thick film on the skin, resulting in discomfort and feeling of a fat film present on the hands. A large amount of ammonia and glycerin makes the base of this means not quite suitable for adding thereto biologically active components, e.g., extracts of vegetable and animal origin. The thick glycerin film hampers the penetration of biologically active components into the skin.

### Disclosure of the Invention

It is an object of the invention to enhance the therapeutic-cosmetic properties of the lotion.

Said object is accomplished by a balanced composition of the lotion which provides softening, cleaning, protective, and tonic action, enables maximum penetration of biologically active components into the skin even at low concentrations of the biologically active substances in the lotion, allows them to act on the skin without causing irritating and allergic effect. Furthermore, the age-related condition of the skin and preference given by definite age groups of people to various odors are taken into account in the elaboration of the formulation of the lotion. This is of no small importance, because the consumer's choice of the lotion is constituted by two factors: the actual therapeutic-cosmetic action on the skin and a pleasant odor (stemming from the presence of essential oils) which must be attractive to the user, since this will stimulate the user to employ the lotion regularly with experiencing positive emotions. Besides, in recent years aromatherapy method has come into use in medicine. In this method aromatic waters comprising a mixture of essential oils are employed as medicaments (S. Althoff, P.N. Williams, et al., "Alternative Medicine", Lincolnwood, Ill.: Publications International Ltd., 1997). When a person inhales the aroma he/or she likes best, this induces not only positive emotions but a curative effect as well.

The claimed lotion for hand skin care comprises glycerin, ethyl alcohol, sodium hydrocarbonate, water, biologically active components (plantain sap and/or kalanchoe sap, and/or liquid extract of eleutherococcus, and/or freshly squeezed lemon juice, and/or pantocrine, and/or aloe sap, and/or hypericum tincture, and/or calendula tincture, and/or freshly squeezed apple juice) and definite essential oils, with the following ratio of the components (in wt.%) :

| | |
|---|---|
| Glycerin | 12.0-17.0 |
| Ethyl alcohol | 8.0-26.0 |
| Sodium hydrocarbonate | 0.08-0.12 |
| Plantain sap, | 1.8-3.8 |
| and/or kalanchoe sap, | |
| and/or liquid extract of eleutherococcus, | |
| and/or freshly squeezed lemon juice, | |
| and/or pantocrine, | |
| and/or aloe sap, | |
| and/or hypericum tincture, | |
| and/or calendula tincture, | |
| and/or freshly squeezed apple juice, | |
| Essential oil | 0.01-0.03 |
| Water | The balance |

When selecting an essential oil and biologically active components, the age determining the specific features of normal physiology of the hand skin and the odor of the essential oil preferred by consumers depending on their age are taken into account. Preference given to a particular essential oil was determined by the following procedure. A group of individuals (males and females separately) were asked to determine the odor they prefer to sense when using a lotion for hand care. Test samples (15 essential oils most often used in cosmetics) were prepared by blending the base with the essential oils i.e., without biologically active additives. The odor of the lotions was determined in accordance with the requirements of GOST 291880.0-91, Section 3.2. The samples were applied to 10x160 mm strips of heavyweight paper, the odor was determined organoleptically. The individuals under test were not told what essential oil was contained in the sample. All age groups which may be potential users of the lotion for hand care were represented in the group. As a result of statistical data treatment it was unexpectedly found that preference to some perfume or other in the lotion formulation was given by clearly outlined age groups of males and females (Table 1).

**Table 1**

| Age (males), years | Preferred perfume | Age (females), years | Preferred perfume |
|---|---|---|---|
| 14-20 | Orange essential oil | 14-19 | Tangerine essential oil |
| 20-38 | Ylang-ylang essential oil | 19-35 | Rosemary essential oil |
| 38-70 | Almond essential oil | 35-70 | Patchouli essential oil |

These essential oils were included into the formulation of the claimed lotion for hand skin care.

The softening, protective and cleaning effects are attained by introducing glycerin, ethyl alcohol, sodium hydrocarbonate into the lotion formulation in definite ratios. Glycerin not only produces softening effect, but also provides a film which protects the skin from adverse environmental effects and moisture loss. Sodium hydrocarbonate reacting with glycerin yields a light-weight soap which provides a light-weight film on the hand skin, which, unlike the glycerin film, is not a fat film and does not cause unpleasant sensations. The glycerin and sodium hydrocarbonate ratio is so selected that maximum cleaning, softening and protective effects are attained.

The ratios of glycerin, ethyl alcohol and sodium hydrocarbonate promote complete penetration of biologically active components into the skin. Besides ethyl alcohol which is a good conductor of biologically active substances, surfactants that form in the reaction of glycerin and sodium hydrocarbonate improve considerably the skin permeability and enhance the penetration of biologically active substances. Ethyl alcohol also provides lotion stability.

Tonic effect of the lotion is provided owing to the biologically active components: plantain sap, kalanchoe sap, liquid extract of eleutherococcus, freshly squeezed lemon juice, pantocrine, aloe sap, hypericum tincture, calendula tincture, freshly squeezed apple juice, and essential oils: tangerine, rosemary, patchouli, orange, ylang-ylang, and almond essential oils.

On the whole the combination of the components provides softening, cleaning, protective and tonic effect which manifests itself in normalization of metabolic processes and stimulation of vital functions of the skin, in preventing its withering.

The lotion preparing process is technologically simple and comprises the following steps.

A vessel is charged with glycerin, a solution of sodium hydrocarbonate is added thereto, and the contents are thoroughly stirred. Separately, another vessel is charged with ethyl alcohol, the ethyl alcohol is blended with one or more of the above-cited biologically active components, and the contents are thoroughly stirred. The solutions thus obtained are combined, intermixed to homogeneous mass, adjusted to the required weight with water, and stirred again.

### The Best Variant of Carrying out the Invention

The invention is illustrated by the following Examples.

### Example 1

Preparing a lotion for females aged from 14 to 19.

15 g of glycerin are weighed out into a vessel, a solution of sodium hydrocarbonate is prepared (0.1 g per 24.9 of drinking water), the liquids are combined and thoroughly intermixed (solution A). 20 g of ethyl alcohol are weighed out into another vessel, 0.02 g of tangerine essential oil is added, and the contents are thoroughly intermixed. Biologically active substances(in the present case, 2.5 g of plantain sap) are added to the resulting solution, and the contents are thoroughly intermixed again (solution B). Solution A and solution B are combined and intermixed. The weight of the mixture is adjusted with drinking water to 100.0 g and thorough stirring is repeated.

The final formulation is presented in Table 2.

**Table 2**

| | |
|---|---|
| Sodium hydrocarbonate | 0.1 g |
| Glycerin | 15 g |
| Plantain sap | 2.5 g |
| Tangerine essential oil | 0.02 g |
| 95% ethyl alcohol | 20 g |
| Drinking water | to 100 g |

### Example 2

Preparing a lotion for females aged from 19 to 35. The components are blended as in Example 1.

The final formulation is presented in Table 3.

**Table 3**

| | |
|---|---|
| Sodium hydrocarbonate | 0.1 g |
| Glycerin | 15 g |
| Kalanchoe sap | 1.0 g |
| Liquid extract of eleutherococcus | 1.0 g |
| Rosemary essential oil | 0.02 g |
| 95% ethyl alcohol | 20 g |
| Drinking water | to 100 g |

### Example 3

Preparing a lotion for females older than 35. The components are blended as in Example 1.

The final formulation is presented in Table 4.

**Table 4**

| | |
|---|---|
| Sodium hydrocarbonate | 0.1 g |
| Glycerin | 15 g |
| Kalanchoe sap | 1.0 g |
| Freshly squeezed lemon juice | 1.5 g |
| Pantocrine | 0.5 g |
| Patchouli essential oil | 0.02 g |
| 95% ethyl alcohol | 20 g |
| Drinking water | to 100 g |

### Example 4

Preparing a lotion for males aged from 14 to 20. The components are blended as in Example 1.

The final formulation is presented in Table 5.

**Table 5**

| | |
|---|---|
| Sodium hydrocarbonate | 0.1 g |
| Glycerin | 15 g |
| Aloe sap | 1.0 g |
| Hypericum tincture | 1.0 g |
| Orange essential oil | 0.02 g |
| 95% ethyl alcohol | 20 g |
| Drinking water | to 100 g |

### Example 5

Preparing a lotion for males aged from 20 to 38. The components are blended as in Example 1.

The final formulation is presented in Table 6.

**Table 6**

| | |
|---|---|
| Sodium hydrocarbonate | 0.1 g |
| Glycerin | 15 g |
| Aloe sap | 1.0 g |
| Calendula tincture | 1.0 g |
| Ylang-ylang essential oil | 0.02 g |
| 95% ethyl alcohol | 20 g |
| Drinking water | to 100 g |

### Example 6

Preparing a lotion for males older than 38. The components are blended as in Example 1.

The final formulation is presented in Table 7.

**Table 7**

| | |
|---|---|
| Sodium hydrocarbonate | 0.1 g |
| Glycerin | 15 g |
| Aloe sap | 1.0 g |
| Freshly squeezed apple juice | 1.0 g |
| Pantocrine | 0.5 g |
| Almond essential oil | 0.02 g |
| 95% ethyl alcohol | 20 g |
| Drinking water | to 100 g |

All the prepared lotions are transparent liquids or slightly opalescing liquids with faint yellowish hues; the hue may pass into greenish (Examples 1, 5) or into orange (Example 4). The pH value of the lotions varies from 7.5 to 8.5.

The use of biologically active additives is not limited to the above-cited Examples. In some cases (for instance, when the condition of the hand skin differs from the physiological norm), other combinations of the biologically active substances, as well as other ratios of the substances constituting the foundation for the hand care lotion, are possible.

Examples 7-9 are presented in Table 8.

**Table 8**

| Amount of components (g) | Example 7 | Example 8 | Example 9 |
|---|---|---|---|
| Sodium hydrocarbonate | 0.09 | 0.15 | 0.1 |
| Glycerin | 13.0 | 16.0 | 15.0 |
| Biologically active sub- | | | |
| stances | | | |
| Plantain sap | - | 0.5 | - |
| Kalanchoe sap | - | 0.3 | - |
| Liquid extract of | | | |
| eleutherococcus | - | 0.4 | - |
| Freshly squeezed lemon | | | |
| juice | - | 0.1 | - |
| Pantocrine | - | 0.6 | - |
| Aloe sap | 1.5 | 0.2 | - |
| Hypericum tincture | - | 0.3 | 1.9 |
| Calendula tincture | - | 0.3 | 1.9 |
| Freshly squeezed apple | | | |
| juice | - | 0.2 | - |

| | | | |
|---|---|---|---|
| Essential oil | orange (0.01) | patchouli (0.03) | rosemary (0.02) |
| 95% ethyl alcohol | 10.5 | 19.5 | 22.0 |
| Drinking water | to 100 | to 100 | to 100 |

The choice of the components indicated in Examples 1-6 is optimal for humans in satisfactory physiological condition, whose actual age corresponds to anthropometric indexes.

The tonic effect of the lotion (according to Example 1) is attained by introducing into its formulation plantain sap which contains bactericide-fungicide-protozoacides, tannins, carotene, polysaccharides, vitamin K, citric and ascorbic acids. Plantain sap promotes healing of scratches and cuts, supports natural processes of cell renewal, collagen and elastin formation. The skin preserves its resilience and elasticity. Tangerine essential oil has a pleasant odor, is a natural source of vitamins, in addition to tonic effect, it produces nutritive and antimicrobial effect on the skin. Optimal combination and ratio of the vegetable components and foundation of the lotion (glycerin, ethyl alcohol, sodium hydrocarbonate) provide maximum penetration of the biologically active substances into the skin.

The tonic effect of the lotion (according to Example 2) is attained by introducing kalanchoe sap and liquid extract of eleutherococcus into the lotion formulation. Kalanchoe sap contains sap contains biogenic stimulants, enzymes, vitamins, produces anti-inflammation effect, promotes cleaning and healing of small wounds and scratches. Extract of eleutherococcus contains sugars, pectins, microelements and provitamins, it is a tonic, normalizes metabolic processes and stimulates vital functions of the skin. Rosemary essential oil has a pleasant odor, is a natural source of vitamins, it produces an antimicrobial effect on the skin, and also inhibits its early withering. Optimal combination and ratio of the vegetable components and the lotion foundation provide maximum penetration of the biologically active substances into the skin.

The tonic effect of the lotion (according to Example 3) is attained by introducing into its formulation kalanchoe sap, freshly squeezed lemon juice and pantocrine. Lemon juice is a natural source of vitamins, it has bleaching, astringent and antiseptic properties, stimulates metabolic processes, biosynthesis, improves cell nutrition and contributes to the regeneration of skin cells. Pantocrine is an adaptogen of natural origin, it contains amino acids, lipids, nucleic acids, microelements. This accounts for its tonic and stimulating effect. Patchouli essential oil, in addition to tonic effect, produces a beneficial effect on dry skin, freshens it up and makes it smooth. Besides a pleasant odor, this essential oil exerts antiviral, bactericidal and anti-inflammatory effect. Optimal combination and ratio of the vegetable components and lotion foundation provide maximum penetration of biologically active substances into the skin.

The tonic effect of the lotion (according to Example 4) is attained by introducing into its formulation aloe sap. Aloe sap contains biogenic stimulants, enzymes, oils and sugars. This sap produces tonic, bactericidal effect, it intensifies skin regeneration processes. An additional effect is provided by introducing hypericum tincture which contains tannins, flavanoids, organic acids, oils and resins. Hypericum tincture exerts astringent, wound-healing, anti-inflammatory and anti-allergic action. Orange essential oil exerts tonic action, is a natural source of vitamins, exerts emollient action on dry skin, freshens it up. In addition to pleasant odor, this oil produces antiviral and anti-inflammatory effect. Optimal combination and ratio of the vegetable components and the lotion foundation provide maximum penetration of the biologically active substances into the skin.

The tonic effect of the lotion (according to Example 5) is attained by introducing into its formulation aloe sap and calendula tincture. Calendula tincture is rich in carotinoids, resins, organic acids polysaccharides and vitamins. Along with tonic effect, calendula tincture exerts pronounced anti-inflammatory and wound-healing action. Ylang-ylang essential oil exerts tonic action, is a natural source of vitamins, exerts emollient action on dry skin, freshens and smoothes it out. Optimal combination and ratio of the vegetable components and the lotion foundation provide maximum penetration of the biologically active substances into the skin.

The tonic effect of the lotion (according to Example 6) is attained by introducing into its formulation aloe sap, pantocrine and freshly squeezed apple juice. Apple juice contains sugars, pectins, organic acids, tannins, essential oils, vitamins, carotene and mineral salts. Along with the tonic effect, it is capable of stimulating metabolic processes, biosynthesis processes, and natural processes of skin regeneration. It improves nutrition of skin cells, refreshing and rejuvenating the skin. Almond essential oil exerts tonic action, is a natural source of vitamins. Along with the tonic effect, almond essential oil exerts emollient action on dry skin, freshens and smoothens it. This essential oil not only has a pleasant odor, it also exerts antiviral and anti-inflammatory action. Optimal combination and ratio of the vegetable components and the lotion foundation provide maximum penetration of the biologically active substances into the skin.

### Industrial Applicability

The claimed formulation of a lotion for hand skin care is advantageous over the existing formulations. 1 to 2 minutes after applying the lotion, a very fine protective film remains on the hands, which film leaves no traces upon contact with objects and does not hamper the user in being occupied with anything. Owing to its low allergenicity, the lotion can be used several times a day and, if required, after each contact with water. For individuals with sensitive skin the lotion makes it possible to preserve the tenderness and comfort of the hand skin, protecting it from harmful environmental effects.

## Claims

1. A lotion for hand skin care, comprising glycerin, ethyl alcohol and water, **characterized in that** it additionally comprises sodium hydrocarbonate, an essential oil, plantain sap and/or kalanchoe sap, and/or liquid extract of eleutherococcus, and/or freshly squeezed lemon juice, and/or pantocrine, and/or aloe sap, and/or hypericum tincture, and/or calendula tincture, and/or freshly squeezed apple juice, with the following ratio of the components (in wt.%) :
| | |
|---|---|
| Glycerin | 12.0-17.0 |
| Ethyl alcohol | 8.0-26.0 |
| Sodium hydrocarbonate | 0.08-0.12 |
| Plantain sap, | 1.8-3.8 |
| and/or kalanchoe sap, | |
| and/or liquid extract of eleutherococcus, | |
| and/or freshly squeezed lemon juice, | |
| and/or pantocrine, | |
| and/or aloe sap, | |
| and/or hypericum tincture, | |
| and/or calendula tincture, | |
| and/or freshly squeezed apple juice, | |
| Essential oil | 0.01-0.03 |
| Water | The balance |

2. A lotion according to claim 1, **characterized in that** it comprises tangerine essential oil for use by females aged from 14 to 19.

3. A lotion according to claim 1, **characterized in that** it comprises rosemary essential oil for use by females aged from 19 to 35.

4. A lotion according to claim 1, **characterized in that** it comprises patchouli essential oil for use by females older than 35.

5. A lotion according to claim 1, **characterized in that** it comprises orange essential oil for use by males aged from 14 to 20.

6. A lotion according to claim 1, **characterized in that** it comprises ylang-ylang essential oil for use by males aged from 20 to 38.

7. A lotion according to claim 1, **characterized in that** it comprises almond essential oil for use by males over 38.
